# EUROPEAN PATENT SPECIFICATION

(11) **EP 0 971 676 B1**
(45) Date of publication and mention of the grant of the patent: **22.12.2004**
(21) Application number: 98907132.9
(22) Date of filing: 23.03.1998
(51) Int. Cl.: A61K 6/00

(54) **DENTURE ADHESIVE COMPOSITIONS**
ZUSAMMENSETZUNG FÜR DIE STABILISATION VON ZAHNPROTHESEN
COMPOSITIONS ADHESIVES POUR PROTHESE DENTAIRE

(30) Priority: 27.03.1997 US 835041; 27.03.1997 US 824940
(43) Date of publication of application: 19.01.2000
(73) Proprietor: THE PROCTER & GAMBLE COMPANY, Cincinnati, Ohio 45202 (US)
(72) Inventor: LIANG, Nong, West Chester, OH 45069 (US); RAJAIAH, Jayanth, Loveland, OH 45140 (US); GILDAY-WEBER, Kimberly, Ann, Cincinnati, OH 45204 (US)
(74) Representative: Clemo, Nicholas Graham
(86) International application number: PCT/IB1998/000433
(87) International publication number: WO 1998/043594

(56) References cited:
- WO-A-92/22280
- WO-A-96/13243

## Description

### BACKGROUND OF THE INVENTION

Ordinary removable dentures, dental plates and the like, comprise teeth mounted in a suitable plate or base. Denture adhesives or stabilizers are used to provide a cushion or gasket between the denture and the gums or tissues and to fill the interstices between the dentures and the gums or tissues.

Denture stabilizing or adhesive compositions can exhibit several deficiencies. Aesthetic deficiencies may include oozing of the adhesive from under the dental plate during insertion and throughout the wearing period and messiness and difficulty of removing the residual adhesive from the mouth and dentures. Additionally, food may become trapped between the denture and the oral cavity of the wearer. Considerable effort has been made over the years to develop denture adhesive compositions with improved holding capabilities which are aesthetically pleasing and easy to use. Both synthetic and natural polymers and gums have been used singly, in combination, and in combination with various adhesives and other materials in an attempt to lessen the deficiencies commonly associated with denture adhesive products.

Lower alkyl vinyl ether-maleic copolymers and salts thereof are known in the art for use in denture adhesive compositions. Such disclosures include: U.S. Patent 3,003,988 to German et al., issued October 10, 1961; U.S. Patent 4,980,391 to Kumar et al., issued December 25, 1990; U.S. Patent 5,073,604 to Holeva et al., issued December 17, 1991; and U.S. Patent 5,525,652 to Clarke, issued June 11, 1996. Layered denture adhesive compositions have also been disclosed in, for example, U.S. Patent 4,880,702 to Homan et al., issued November 14, 1989 and European Patent Application 0,353,375 to Altwirth, published February 7, 1990. Despite the above-noted technologies, as well as others, a need still exists for denture stabilizing compositions providing improved hold and aesthetics.

In accordance with the present invention, it has been discovered that denture adhesive compositions comprising ferric iron and divalent and/or monovalent metal partial salts of lower alkyl vinyl ether-maleic acid copolymers, and optionally at least one non-adhesive self-supporting layer, provide superior denture stability and retention over a significantly longer period of time versus conventional denture adhesives. Specifically, the compositions exhibit higher resistance to salivary washout while maintaining the same or better denture hold as conventional denture adhesives. This added resistance to salivary washout translates to longer denture hold and stability.

### SUMMARY OF THE INVENTION

The present invention relates to a denture adhesive composition comprising partial salts of a C₁-C₄ alkyl vinyl ether-maleic acid copolymer wherein the partial salt contains a cationic salt function consisting essentially of from 0.1% to 10% ferric iron cations; and from 0.1% to 75% of divalent and/or monovalent metal cations selected from the group consisting of zinc, calcium, magnesium, potassium, sodium, ammonium, and mixtures thereof, wherein strontium cations are not used in combination with zinc cations. These compositions may optionally comprise at least one non-adhesive self-supporting layer.

### DETAILED DESCRIPTION OF THE INVENTION

The denture adhesive compositions of the present invention comprise a partial salt of a C₁-C₄ alkyl vinyl ether-maleic acid copolymer having a cationic salt function consisting essentially of ferric iron cations and also one or more of divalent and/or monovalent metal cations. The adhesive compositions may in the form of a powder which is sprinkled on a dental prosthesis, moistened and then inserted into the oral cavity. The compositions may also be combined with various conventional delivery vehicles to form liquids or pastes which are applied to a dental prosthesis and inserted into the oral cavity.

The compositions may also comprise a non-adhesive self-supporting layer. The adhesive compositions are thoroughly moistened and applied to dentures. The attachment of the adhesive to the non-adhesive self-supporting layer provides a composition which is peeled from the dentures upon their removal. A detailed description of essential and optional components of the present invention is given below.

### Lower Alkyl Vinyl Ether-Maleic Partial Salt Polymer

The lower alkyl vinyl ether-maleic acid ("AVE/MA") copolymer consists essentially of the repeated structural unit: 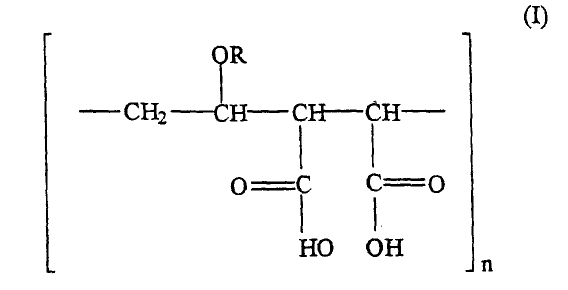 wherein R represents a C1 to C4 alkyl radical, n is an integer greater than one representing the number of repeated occurrences of the structural unit in a molecule of the copolymer. In characterizing the copolymer, n is large enough such that the specific viscosity of the copolymer is larger than 1.2, the specific viscosity being determined in methyl ether ketone at 25°C.

Lower alkyl vinyl ether maleic polymers are readily obtained by copolymerizing a lower alkyl vinyl ether monomer, such as methyl vinyl ether, ethyl vinyl ether, divinyl ether, propyl vinyl ether, isobutyl vinyl ether and the like, with maleic anhydride to yield the corresponding lower alkyl vinyl ether-maleic anhydride copolymer which is readily hydrolyzable to the acid copolymer. In general, the resulting copolymer is a 1:1 copolymer. Both anhydride and acid forms are also available from commercial suppliers. For example, ISP Technologies Inc. ("ISP"), Wayne, New Jersey, provides both the polymeric free acid form (I) and the corresponding anhydride form under its "GANTREZ" trademark as the "GANTREZ S Series" and "GANTREZ AN Series", respectively. In the former acid series, the GANTREZ S-97 is particularly suitable, and, in the latter anhydride series, the GANTREZ AN-149 (specific viscosity of 1.5 to 2.5) the GANTREZ AN-169 (specific viscosity of 2.6 to 3.5) and the GANTREZ AN-179 (specific viscosity of 3.5 to 5.0) copolymers are particularly suitable. These copolymers are described in greater detail in U.S. Patent 5,395,867 to Prosise, issued 3/7/95. When the anhydride copolymer dissolves in water, the anhydride linkage is cleaved so that the highly polar, polymeric free acid (I) is formed. Accordingly, the anhydride form, which is relatively less expensive than the acid form, may be used as a convenient and cheaper precursor for the acid. Elevated temperatures may be advantageously employed to enhance the rate of anhydride-to-acid hydrolysis.

The C₁-C₄ alkyl vinyl ether-maleic acid ("AVE/MA") polymers useful in the present invention are partial copolymer salts. Such salts comprise a cationic salt function. The cationic salt function contains ferric iron and also one or more metal cations selected from the group consisting of divalent cations, monovalent cations, and mixtures thereof. Divalent metal cations include zinc, strontium (but not used in combination with zinc), calcium, magnesium and mixtures thereof. Monovalent metal cations include sodium, potassium, ammonium, and mixtures thereof. Partial salts of lower alkyl vinyl ether-maleic acid polymers are also described in U.S. Patent 5,073,604 to Holeva et al., issued 12/17/91; U.S. Patent 5,204,414 to Pelah et al., issued 4/20/93; and U.S. Patent 5,525,652 to Clarke, issued 6/11/96.

The copolymer salts may be mixed or unmixed or both. The term "unmixed polymer salts" as used herein refers to salts of lower alkyl vinyl ether-maleic polymers wherein the cations are unmixed with any other ester functions or nonidentical cations on the same polymer, the remaining carboxyl groups being unreacted.

The term "mixed polymer salts" as used herein refers to salts of the lower alkyl vinyl ether-maleic polymers where different cations are mixed on the same polymer with each other or with other ester functions. Preferred are mixed polymer salts containing zinc and calcium cations.

Partial copolymer salts comprising ferric iron cations can be prepared by the interaction of the AVE/M anhydride/acid copolymers with ferric compounds, in the form of a salt, such as ferric sulfate pentahydrate. Partial copolymer salts comprising divalent and/or monovalent metal cations can be prepared by the interaction of the AVE/M anhydride/acid copolymers with metal cation (such as zinc, strontium, calcium, magnesium, sodium, potassium, or ammonium) compounds either in the form of a base or a salt; such as, for example, the hydroxide, acetate, halide, lactate, etc. in an aqueous medium. In a preferred embodiment, the oxide of zinc and the hydroxide of calcium are utilized. Since zinc hydroxide is not commercially available, its use as a reactant is readily and more economically accomplished by employing an aqueous slurry of particular zinc oxide which, although practically insoluble in water, provides hydration to zinc hydroxide on the particulate surface.

The sum total of the metal cations in the resultant partial salt of AVE/MA copolymers should be sufficient to give a neutralization ranging from 0.1% to 75% of divalent and/or monovalent metal cations, of the initial carboyxl groups reacted. The resulting partial salt copolymer contains free acid in the range of from 5% to 50%.

In preferred partial copolymer salts, the cationic salt function contains ferric iron from 0.01% to 10%, preferably from 0.05% to 5%, and most preferably from 0.1% to 3%, of the initial carboxyl groups reacted; zinc from 10% to 65%, preferably from 5% to 45%, and most preferably from 10% to 30%, of the initial carboxyl groups reacted; and calcium from 10% to 75%, preferably from 25% to 60%, and most preferably from about 40% to about 60%, of the total initial carboxyl groups reacted. Also preferred is sodium from 1% to 20%, preferably from 1% to 15%, and most preferably from 1% to 10%, of the total initial carboxyl groups reacted; and strontium from 10% to 75%, preferably from 25% to 60%, and most preferably from 40% to 60%, of the total initial carboxyl groups reacted.

Cations that form toxic, irritating or contaminating by-products should be avoided, or special precautions and treatment provided to assure the removal and absence of such by-products from the polymeric salt end-product. The particular compound used should be substantially pure to assure obtaining a substantially pure, substantially off-white copolymeric salt end-product. The partial salt copolymers are utilized in the present composition in an amount of at least 10 percent and more preferably in amount of at least 20 percent, by weight of the adhesive composition.

### Reducing Agent

The present invention can also comprise the use of a reducing agent. The reducing agent aids in removal of the denture from the oral cavity after application of the present ferric iron/divalent and/or monovalent metal cation partial copolymer salts to the denture. While not to be bound by theory, it is believed that the reducing agent reduces ferric iron to ferrous iron, thus reducing the adhesive properties of the partial salt copolymer and facilitating removal of the denture(s). The preferred reducing agent for use herein is ascorbic acid and its water soluble salts.

The reducing agent may also be used in combination with a chelating agent. Preferred chelating agents include citrate, tartrate, lactate, and the like. The reducing agent and/or chelating agent may also be delivered in a composition by carriers known in the art which are safe for oral administration (i.e., non-toxic and approved for use in humans). Such carriers include surfactants, and solvents.

The reducing agent and/or chelating agents are used in safe and effect amounts. The term "safe and effective amount", as used herein, means an amount sufficient to aid in releasing the denture hold in the oral cavity without toxicity to the user, damage to oral tissue, and alteration of the denture material. Thus, a denture wearer applies the ferric iron/metal(s) cation partial copolymer salt adhesive composition to dentures and inserts them into the oral cavity. When removal is desired, the wearer swishes in the mouth, a denture releasing composition comprising a reducing agent and/or chelating agents and suitable solvent(s) which aids in releasing the denture hold.

### Non-Adhesive Self-Supporting Layer

The present denture adhesive compositions comprise at least one non-adhesive self-supporting layer. The non-adhesive self-supporting layer is characterized by its ability to maintain strength and provide integrity for the adhesive composition in the presence of water and/or saliva. The non-adhesive self-supporting layer may include such materials as polyester, polypropylene, nylon, rayon, polyethylene oxide, cellulose acetate, cellulose derivatives, cloth, fibrous fleece, paper, plastic, leather, microcrystalline wax, synthetic fibers, natural fibers, and mixtures thereof Preferred are cellulose derivatives, polyester, polypropylene, nylon, rayon, cloth, paper, microcrystalline wax, and mixtures thereof. Most preferred are polyester, polypropylene, rayon, nylon, cloth and paper.

The non-adhesive self-supporting layer may be in any physical form suitable for providing strength and/or integrity to the present adhesive compositions. Such physical forms include non-woven, woven, continuous, chopped, and combinations thereof. In addition, the non-adhesive self-supporting layer may be formed by any process commonly known in the art. Such processes include un-bonded, spraybonded, spun-bonded, needle-punched, carded, thermal bonded hydroentangled, meltblown, aperture print bonded, needled, wet-laid, dry-laid, and combinations thereof.

### Additional Adhesive Components

The present invention compositions may also include other adhesive components. These adhesive components, if present, are used in safe and adhesively effective amounts. The term "safe and adhesively effective amount" as used herein means an amount sufficient to provide adherence of a dental prosthesis to the palate and ridge of the oral cavity without toxicity to the user, damage to oral tissue, and alteration of the denture material.

Suitable adhesive components include a water-soluble hydrophilic colloid or polymer having the property of swelling upon exposure to moisture to form a mucilaginous mass. Such adhesive materials include natural gums, synthetic polymeric gums, adhesive materials commonly employed in denture stabilizing compositions and compatible with the subject AVE/MA copolymers, synthetic polymers, mucoadhesive polymers, hydrophilic polymers, saccharide derivatives, cellulose derivatives, and mixtures thereof. Examples of such materials include karaya gum, guar gum, gelatin, algin, sodium alginate, tragacanth, chitosan, polyethylene glycol, acrylamide polymers, carbopol, polyvinyl alcohol, polyamines, polyquarternary compounds, polybutenes, silicones, ethylene oxide polymers, polyvinylpyrrolidone, cationic polyacrylamide polymers.

Preferred are cellulose derivatives such as methylcellulose, sodium carboxymethylcellulose, hydroxyethylcellulose, hydroxypropylcellulose, hydroxypropylmethylcellulose, carboxymethylcellulose. Most preferred are carboxymethylcellulose, polyethylene glycol, polyethylene oxide, karaya gum, sodium alginate, chitosan, polyvinyl alcohol, and mixtures thereof. In general, the other adhesive components may be present at a level of from 0% to 70%, preferably from 10% to 50%, and most preferably from 20% to 40%, by weight of the composition.

### Other Ingredients

One or more toxicologically-acceptable plasticizers may also be included in the present compositions. The term "toxicologically-acceptable", as used herein, is used to describe materials that are suitable in their toxicity profile for administration to humans and/or lower animals. Plasticizers that may be used in the present compositions include dimethyl phthalate, diethyl phthalate, dioctyl phthalate, glycerin, diethylene glycol, triethylene glycol, Igepal, Gafac, sorbitol, tricresyl phosphate, dimethyl sebacate, ethyl glycolate, ethylphthalyl ethyl glycolate, o- and p-toluene ethyl sulfonamide, and mixtures thereof. Plasticizers may be present at a level of from 0% to 70%, preferably from 0.1% to 30%, by weight of the compositions.

The denture adhesive compositions may also be used as a denture adhesive and/or bioadhesive and comprise one or more therapeutic actives suitable for mucosal or topical administration. The phrase "suitable for mucosal or topical administration", as used herein, describes agents which are pharmacologically active when absorbed through internal mucosal surfaces of the body such as the oral cavity, or applied to the surfaces of the skin. Therapeutic actives may be present at a level of from 0% to 70%, by weight of the composition.

Therapeutic actives that are useful in these compositions include antimicrobial agents such as iodine, sulfonamides, bisbiguanides, or phenolics; antibiotics such as tetracycline, neomycin, kanamycin, metronidazole, or clindamycin; anti-inflammatory agents such as aspirin, acetaminophen, naproxen and its salts, ibuprofen, ketorolac, flurbiprofen, indomethacin, cimetidine, eugenol, or hydrocortisone; dentinal desensitizing agents such as potassium nitrate, strontium chloride or sodium fluoride; anesthetic agents such as lidocaine or benzocaine; anti-fungals; aromatics such as camphor, eucalyptus oil, and aldehyde derivatives such as benzaldehyde; insulin; steroids; and anti-neoplastics. It is recognized that in certain forms of therapy, combinations of these agents in the same delivery system may be useful in order to obtain an optimal effect. Thus, for example, an antimicrobial and an anti-inflammatory agent may be combined in a single delivery system to provide combined effectiveness.

Other suitable ingredients include silicon dioxide, colorants, preservatives such as methyl and propyl parabens; thickeners, and polyethylene glycol; and delivery vehicles such as liquid petrolatum, petrolatum, mineral oil and glycerin. Preferred are polyethylene glycol, silicon dioxide, and petrolatum. Colorants, preservatives, thickeners and delivery vehicles may be present at levels of from 0% to 20%, by weight of the composition.

The compositions of the present invention may also include one or more components which provide flavor, fragrance, and/or sensate benefit. Suitable components include natural or artificial sweetening agents, menthol, menthyl lactate, wintergreen oil, peppermint oil, spearmint oil, leaf alcohol, as well as coolants 3-1-menthoxypropane-1,2-diol and paramenthane carboxyamide agents such as N-ethyl-p-menthane-3-carboxamide which is described in U.S. Patent 4,136,163 to Watson et. al.,. These agents may be present at a level of from 0% to 50%, by weight of the composition.

### Process for Preparation of the Composition

The present adhesive copolymers can be prepared by any of the methods or combination of methods which follow. The term mixture, as used herein, refers to a solution, slurry, or suspension.

The lower alkyl vinyl ether maleic anhydride copolymers can be obtained either from commercial suppliers under the trade names disclosed previously or by copolymerization of a lower alkyl vinyl ether monomer with maleic anhydride to yield the corresponding lower alkyl vinyl ether-maleic anhydride copolymer which is readily hydrolyzable to the acid copolymer. Processes for the preparation of partial AVE/MA copolymer salts is also described in U.S. 5,073,604 to Holeva, issued 12/17/91.

The AVE/MA copolymer is hydrolyzed and neutralized in an aqueous mixture or slurry of one or more divalent and/or monovalent metal bases by heating the copolymer/base mixture to a temperature ranging from about 45°C to about 100°C. Reaction of the partial AVE/MA copolymer salt with ferric iron cations is obtained through addition of ferric iron salts to the hydrolyzed and neutralized partial salt of the AVE/MA copolymer. Completion of the reaction with ferric iron cations is indicated by an increase in viscosity to stabilization. Alternatively, ferric iron salts may be blended with the copolymer/metal base mixture prior to the hydrolysis and neutralization reactions.

The resulting slurry or solution is transferred to shallow stainless steel drying trays and placed in a forced air mechanical convection oven at 60°C for a time sufficient to evaporate the reaction medium (water) and remove water from the copolymer (about 18-24 hours). Alternatively, the resulting slurry or solution can be drum-dried. After drying, the polymer forms brittle flakes which can easily be peeled off from the trays or drum surface and ground to a fine powder as desired.

In compositions comprising a non-adhesive self-supporting layer, the adhesive components (partial salt copolymers and other adhesive components, if present) may be coated on the non-adhesive self-supporting layer using various methods. These include: (a) wetting the non-adhesive self-supporting layer with water, uniformly sifting the adhesive component powder(s) onto the wet layer and then rewetting the layer with water; (b) dissolving the adhesive component(s) in water and/or other solvent(s) and coating the resulting mixture onto the layer; (c) coating the layer with the mixture produced during Gantrez® processing; (d) incorporating the adhesive component(s) into the layer as the layer is being formed; and (e) dissolving the adhesive component(s) in water and/or other solvent(s), wetting/coating the resulting mixture onto the layer, and uniformly sifting one or more adhesives in powder form onto the wet/coated layer and optionally re-coating/re-wetting the layer with the mixture and/or water; (f) the method of step (e) repeated multiple times; and (g) any combination of the methods in (a) through (f) above.

As disclosed above, the adhesive components may be dissolved in water and/or other solvents and the resulting mixture coated onto the layer. Solvents for the AVE/MA polymers include water and/or alcohols such as methanol, propanol, isopropanol, ethanol, butanol, 1,4-butanediol, cyclohexanol, and diethylene glycol; ethers or ether alcohols such as tetrahydrofuran, ethylene glycol monomethyl ether, diethylene glycol monomethyl ether, dioxane, and ethyl ether; esters such as methyl acetate, ethyl acetate and sec-butyl acetate; aldehydes, ketones or ketone-alcohols such as benzaldehyde, formaldehyde solution, methyl ethyl ketone, diacetone alcohol, acetone, cyclohexanone, mesityl oxide, and methyl isobutyl ketone; lactams or lactones such as N-methyl-2-pyrrolidone, N-vinyl-2-pyrrolidone, 2-pyrrolidone, and butyrolactone; hydrocarbons such as benzene, toluene, xylene, hexane, mineral spirits, mineral oil, and gasoline; chlorinated hydrocarbons such as carbon tetrachloride, chlorobenzene, chloroform, ethylene dichloride, methylene chloride; nitroparaffins such as nitroethane, and nitromethane; mercaptans such as thiophenol and 2-mercapto-1-ethanol; and others such as acetic acid, pyridine and dimethyl formamide.

Preferred solvents for the AVE/MA polymers are water, methanol, propanol, isopropanol, tetrahydrofuran, methyl acetate, benzaldehyde, formaldehyde solution, methyl ethyl ketone, diacetone alcohol, N-methyl-2-pyrrolidone, N-vinyl-2-pyrrolidone, dimethyl formamideand mixtures thereof. Compounds commonly used as plasticizers can also be used as solvents for the AVE/MA polymers. Such plasticizers include dimethyl phthalate, diethyl phthalate, dioctyl phthalate, glycercin, diethylene glycol, triethylene glycol., Igepal® CO-630, Gafac ® RE-610, Sorbitol, tricresyl phosphate, dimethyl sebacate, ethyl glycolate, ethylphthalyl ethyl glycolate, and p-toluene ethyl sulfonamide.

Solvents for other adhesives such as carboxymethylcellulose ("CMC") which may be optionally included in the adhesive compositions include mixtures of water and water-miscible solvents such as ethanol and acetone. Solutions of low concentration can be made with up to 40% acetone and/or 50% alcohol. Other solvents which made be used include ethanolamines; ethylene glycol; glycerol; 1,2,6-hexanetriol; mono-, di-, and triacetin; 1,5-pentanediol; polyethylene glycol (molecular weight 600 or less); propylene glycol; and trimethylolpropane.

When the adhesive compositions are prepared by dissolving the adhesive component(s) in water and/or other solvents, various embodiments of the process includes: dissolving AVE/MA adhesives in one or more of the solvents for AVE/MA polymers; dissolving an optional adhesive in a suitable solvent and coating the resulting mixture onto the non-adhesive self-supporting layer and then optionally sifting one or more adhesives onto the coated layer. Coating the layer can be achieved by techniques commonly known in the art including extrusion, doctor blading, spraying, dipping, etc.

After the adhesive component(s) has been deposited on a layer by one of the means described above, the layer is then dried. Next, the denture adhesive composition is mechanically softened by running it through a ring-roller or micro-cracker or any other suitable means. The composition is then pressed smooth in a hydraulic press or flat-roller or other suitable means. The composition is then die-cut into denture shapes. These shapes may facilitate application of the composition to the dentures.

The following examples further describe and demonstrate embodiments within the scope of the present invention. The examples are given solely for the purpose of illustration.

### EXAMPLE I

Into a 2 liter resin reaction kettle, equipped with a high torque mixer with a built-in viscosity monitor, which contains 1484.95 grams of purified water, USP at room temperature, add 0.048 grams of ferric sulfate pentahydrate (Fe₂(SO₄)₃•5H₂O). Mix for about 5 minutes at 300 rpm with a paddle stirring element. Slowly add 15.00 grams of 65% neutralized mixed calcium (47.5%) and zinc (17.5%) partial salt of methyl vinyl ether/maleic acid ("MVE/MA") copolymer made from Gantrez® AN169 at an appropriate speed such that the salt is dispersed before it becomes fully hydrated. Heat the resin reaction kettle with a temperature controlled water bath to 90°C and maintain the reaction temperature between 85°C and 95°C at a constant agitation rate of 300 rpm until no clumps are visible (1 to 3 hours). The reaction batch is a homogeneous dispersion of water insoluble polymer.

Transfer the resulting slurry to shallow stainless steel drying trays. Dry in a forced air mechanical convection oven at 60°C for a sufficient time to evaporate the reaction medium (water) and remove the water from the polymer (about 18 to 24 hours). After drying, the sticky polymer forms brittle flakes easily peeling off from the drying trays. Grind the flakes to a fine powder in a milling apparatus such as speed-rotor mill with an appropriate screen to define the mean particle size and its distribution. A 0.12 mm or 0.08 mm screen is preferred. The resulting adhesive copolymer yields a 65% neutralized [mixed calcium (47.5%), zinc (17.5%)] and ferric iron (0.4%) partial salt of MVE/MA copolymer.

In compositions comprising at least one non-adhesive self-supporting layer, wet 58" by 20" of the non-adhesive self-supporting layer with water. Uniformly coat 150 grams of adhesive components (90g CaZn/Fe partial salt MVE/MA copolymer and 60g carboxymethylcellulose) onto the layer and rewet the layer with water. Dry the layer. Mechanically soften the denture adhesive composition by ring-roller, and then smooth the composition on a hydraulic press. Cut the composition into denture-shaped wafers. Moisten the wafers and apply to the dentures. This wafer is peelable from the denture and forms a sticky seal that holds the dentures in place, does not ooze, and aids in preventing food from sticking between the dentures and gums.

Variations in the amount of ferric iron useful herein include the following:

| | Fe₂(SO₄)₃•5H₂O (gram) | Fe(III)% |
|---|---|---|
| A | 0.024 | 0.2 |
| B | 0.048 | 0.4 |
| .C | 0.072 | 0.6 |
| D | 0.096 | 0.8 |
| E | 0.120 | 1.0 |

### EXAMPLE II

Into a 2 liter resin reaction kettle, equipped with a high torque mixer with a built-in viscosity monitor, which contains 1429.50 grams of purified water, USP at room temperature, add 0.60 grams of ferric sulfate pentahydrate (Fe₂(SO₄)₃•5H₂O). Mix for about 5 minutes at 300 rpm with a paddle stirring element. Slowly add 12.30 grams of calcium hydroxide until the solid is well dispersed. Then slowly add 57.60 grams of MVE/MA copolymer or Gantrez® AN169 until the solid is well dispersed. Heat the resin reaction kettle with a temperature controlled water bath to 90°C and maintain the reaction temperature between 85°C and 95°C at a constant agitation rate of 300 rpm. Transparent clarity of the reaction batch and an increase in pH to a stable value, (typically around pH 4.9 measured in an aliquot of 1:10 (v/v) dilution) indicates the completion of hydrolysis and neutralization reactions.

Repeat the drying and milling procedures described in Example I. The resulting adhesive copolymer yields a 45% neutralized [Ca (45%)] and Fe(III) (1.0%) partial salt of MVE/MA copolymer. The procedures for preparing a non-adhesive self-supporting layer, as described in Example I, may also be repeated.

### EXAMPLES III-VII

| Example # | Adhesive Component | Non-Adhesive Self-Supporting Layer |
|---|---|---|
| III | 47.5 Ca/17.5 Zn/0.4 Fe MVE/MA^{(b)} | |
| | +CMC^{(a)} | Non-woven polyester |
| IV | 47.5 Ca/17.5 Zn/0.4 Fe MVE/MA^{(b)} | |
| | +CMC^{(a)} | Polypropylene |
| V | 45 Ca/1.0 Fe MVE/MA^{(c)} +CMC^{(a)} | Non-woven rayon |
| VI | 27.5 Ca/20.0 Sr/17.5 Zn/0.5 Fe MVE/MA^{(d)} | |
| | +CMC^{(a)} | Cloth |
| VII | 45 Ca/1.0 Fe MVE/MA/PEG^{(c)}+CMC^{(a)} | Paper |

| | | |
|---|---|---|
| ^{(a)} Carboxy methyl cellulose. | | |
| ^{(b)} Methyl vinyl ether-maleic acid partial salt neutralized with 47.5% calcium and 17.5% zinc, and 0.4% ferric iron. | | |
| ^{(c)} Methyl vinyl ether-maleic acid partial salt neutralized with 45% calcium and 1.0% ferric iron. | | |
| ^{(d)} Methyl vinyl ether-maleic acid partial salt neutralized with 47.5% calcium, 20.0% strontium, 17.5% zinc, and 0.5% ferric iron. | | |

The adhesive component in Examples III-VII may also include 0.1 to 15 grams of silicon dioxide.

Examples III-VII are prepared as follows. Wet 58" by 20" of the non-adhesive self-supporting layer with water. Uniformly coat 150 grams of the adhesive component (90g MVE/MA/PEG partial salt copolymer and 60g CMC) onto the layer and rewet the layer with water. Dry the layer. Mechanically soften the denture adhesive composition by ring-roller, and then smooth the composition on a hydraulic press. Cut the composition into denture-shaped wafers. Moisten the wafers and apply to the dentures. This wafer is peelable from the denture and forms a sticky seal that holds the dentures in place, does not ooze, and aids in preventing food from sticking between the dentures and gums.

### EXAMPLE VIII

Denture stabilizing compositions in powder form can be made by blending together the following ingredients:

| | | % w/w |
|---|---|---|
| A | Carboxymethylcellulose Sodium | 58.9 |
| | 47.5% Ca, 17.5% Zn, 0.4% Fe(III) Partial Salt of MVE/MA Copolymer | 40.0 |
| | Silicon Dioxide, Colloidal | 1.0 |
| | Peppermint Flavor Oil | 0.1 |
| B | Karaya Gum | 40.0 |
| | Carboxymethylcellulose Sodium | 28.9 |
| | 47.5% Ca, 17.5% Zn, 0.4% Fe(III) Partial Salt of MVE/MA Copolymer | 30.0 |
| | Silicon Dioxide, Colloidal | 1.0 |
| | Peppermint Flavor Oil | 0.1 |
| C | Carboxymethylcellulose Sodium | 50.0 |
| | 45.0% Ca, 1.0% Fe(III) Partial Salt of MVE/MA Copolymer | 48.9 |
| | Silicon Dioxide, Colloidal | 1.0 |
| | Peppermint Flavor Oil | 0.1 |

### EXAMPLE IX

Denture stabilizing compositions in cream form can be made by blending together the following ingredients:

| | A | B | C |
|---|---|---|---|
| | %, w/w | %, w/w | % w/w |
| White Mineral Oil | 24.82 | 24.82 | 24.82 |
| Petrolatum | 19.02 | 19.02 | 19.08 |
| Carboxymethylcellulose Sodium | 22.00 | 32.00 | 12.00 |
| Silicon Dioxide, Colloidal | 1.10 | 1.10 | 1.10 |
| Colorant (oil soluble red color dispersion) | 0.06 | 0.06 | - |
| 47.5% Ca, 17.5% Zn, 0.4% Fe(III) Partial Salt of | 33 | - | - |
| MVE/MA Copolymer | | | |
| 27.5% Ca, 20.0% Sr, 17.5% Zn, 0.5% Fe(III) Partial | - | 23 | - |
| Salt of MVE/MA Copolymer | | | |
| 45.0% Ca, 1.0% Fe(III) Partial Salt of MVE/MA | - | - | 43 |
| Copolymer | | | |

Flavor oil may be incorporated into the composition at 0.1% to 0.5% (w/w) level.

### EXAMPLE X

Denture stabilizing compositions in liquid form can be made by blending together the following ingredients:

| | A | B | C |
|---|---|---|---|
| | %, w/w | %, w/w | % w/w |
| White Mineral Oil | 48.82 | 50.82 | 46.88 |
| Petrolatum | 5.02 | 3.02 | 7.02 |
| Carboxymethylcellulose Sodium | 18.00 | 26.00 | 8.00 |
| Silicon Dioxide, Colloidal | 1.10 | 1.10 | 1.10 |
| Colorant (oil soluble red color dispersion) | 0.06 | 0.06 | - |
| 47.5% Ca, 17.5% Zn, 0.4% Fe(III) Partial Salt of | 27 | - | - |
| MVE/MA Copolymer | | | |
| 27.5% Ca, 20.0% Sr, 17.5% Zn, 0.5% | - | 19. | - |
| Fe(III) Partial Salt of MVE/MA Copolymer | | | |
| 45.0% Ca, 1.0% Fe(III) Partial Salt of | - | - | 37 |
| MVE/MA Copolymer | | | |

Flavor oil may be incorporated into the composition at 0.1% to 0.5% (w/w) level.

## Claims

1. A denture adhesive composition comprising a partial salt of a C₁ - C₄ alkyl vinyl ether-maleic acid copolymer wherein the partial salt comprises a cationic salt function consisting essentially of:
a) from 0.01% to 10% of ferric iron cations; and
b) from 0.1% to 75% of divalent and/or monovalent metal cations selected from zinc, calcium, magnesium, potassium, sodium, ammonium, and mixtures thereof,
wherein strontium cations are not used in combination with zinc cations.

2. The denture adhesive composition according to Claim 1 wherein the divalent and/or monovalent metal cations are selected from strontium, calcium, magnesium, potassium, sodium, ammonium, and mixtures thereof.

3. The denture adhesive composition according to Claim 1 or Claim 2 wherein the metal cations are divalent metal cations selected from zinc, calcium, magnesium and mixtures thereof.

4. The denture adhesive composition according to any of Claims 1 to 3 comprising from 0.5% to 5% ferric iron cations.

5. The denture adhesive composition according to any of Claims 1 to 4 further comprising an additional adhesive component selected from natural gums, synthetic polymers, mucoadhesive polymers, hydrophilic polymers, natural polymers, saccharide derivatives, cellulose derivatives, and mixtures thereof.

6. The denture adhesive composition according to any of Claims 1 to 5 comprising an adhesive cellulose derivative selected from hydroxyethylcellulose, hydroxypropylcellulose, hydroxypropylmethyl cellulose, sodium carboxymethylcellulose, and mixtures thereof.

7. The denture adhesive composition according to any of Claims 1 to 6 further comprising a flavour selected from menthol, menthyl lactate, peppermint oil, spearmint oil, leaf alcohol, p-menthane carboxamides, and mixtures thereof.

8. The denture adhesive composition according to any of Claims 1 to 7 comprising from 10% to 50% carboxymethylcellulose, by weight of the composition, wherein the cationic salt function consists essentially of:
a) from 0.1% to 3% of ferric iron cations;
b) from 10% to 65% of zinc; and
c) from 10% to 75% calcium,
of the initial carboxyl groups reacted.

9. The denture adhesive composition according to any of Claims 1 to 8 further comprising at least one non-adhesive self-supporting layer selected from polyester, polypropylene, nylon, rayon, polyethylene oxide, cellulose acetate, cellulose derivatives, cloth, fibrous fleece, paper, plastic, leather, microcrystalline wax, synthetic fibers, natural fibers, and mixtures thereof.

10. A process for the preparation of the denture adhesive composition according to any of Claims 1 to 9 comprising the steps of:
a) partially neutralizing a C₁ - C₄ alkyl vinyl ether-maleic acid copolymer with one or more metal cations selected from divalent metal cations, monovalent metal cations, and mixtures thereof;
b) adding ferric iron cations to the partially neutralized copolymer to form a partial copolymer salt composition; and
c) drying the composition to form a powder.

11. The process according to Claim 10 further comprising the steps of:
a) mixing the powder with water and/or one or more solvents to create a mixture; and
b) applying the mixture onto a non-adhesive self-supporting layer.

## Patentansprüche

1. Gebiss-Haftzusammensetzung, umfassend ein Partialsalz eines C₁-C₄-Alkylvinylether-Maleinsäure-Copolymeren, wobei das Partialsalz eine kationische Salzfunktion umfasst, bestehend im wesentlichen aus:
a) 0,01% bis 10% Eisen(III)-Kationen; und
b) 0,1% bis 75% zweiwertige und/oder einwertige Metallkationen, gewählt aus Zink, Calcium, Magnesium, Kalium, Natrium, Ammonium und Mischungen hiervon,
wobei Strontiumkationen nicht in Kombination mit Zinkkationen verwendet werden.

2. Gebiss-Haftzusammensetzung nach Anspruch 1, wobei die zweiwertigen und/oder einwertigen Metallkationen gewählt sind aus Strontium, Calcium, Magnesium, Kalium, Natrium, Ammonium und Mischungen hiervon.

3. Gebiss-Haftzusammensetzung nach Anspruch 1 oder Anspruch 2, wobei die Metallkationen zweiwertige Metallkationen sind, gewählt aus Zink, Calcium, Magnesium und Mischungen hiervon.

4. Gebiss-Haftzusammensetzung nach mindestens einem der Ansprüche 1 bis 3, umfassend 0,5% bis 5% Eisen(III)-Kationen.

5. Gebiss-Haftzusammensetzung nach mindestens einem der Ansprüche 1 bis 4, umfassend weiterhin eine zusätzliche Haftkomponente, gewählt aus natürlichen Gummen, synthetischen Polymeren, mucoadhäsiven Polymeren, hydrophilen Polymeren, natürlichen Polymeren, Saccharidderivaten, Cellulosederviaten und Mischungen hiervon.

6. Gebiss-Haftzusammensetzung nach mindestens einem der Ansprüche 1 bis 5, umfassend ein adhäsives Cellulosederivat, gewählt aus Hydroxyethylcellulose, Hydroxypropylmethylcellulose, Natriumcarboxymethylcellulose und Mischungen hiervon.

7. Gebiss-Haftzusammensetzung nach mindestens einem der Ansprüche 1 bis 6, umfassend weiterhin einen Aromastoff, gewählt aus Menthol, Menthyllactat, Pfefferminzöl, Spearmintöl, Blattalkohol, p-Menthancarboxamiden und Mischungen hiervon.

8. Gebiss-Haftzusammensetzung nach mindestens einem der Ansprüche 1 bis 7, umfassend 10% bis 50% Carboxymethylcellulose, bezogen auf Gewicht der Zusammensetzung, wobei die kationische Salzfunktion im wesentlichen besteht aus:
a) 0,1% bis 3% Eisen(III)-Kationen;
b) 10% bis 65% Zink; und
c) 10% bis 75% Calcium,
der reagierten anfänglichen Carboxylgruppen.

9. Gebiss-Haftzusammensetzung nach mindestens einem der Ansprüche 1 bis 8, umfassend weiterhin mindestens eine nichtadhäsive, selbsttragende Schicht, gewählt aus Polyester, Polypropylen, Nylon, Rayon, Polyethylenoxid, Celluloseacetat, Cellulosederivaten, Stoff, Faservlies, Papier, Kunststoff, Leder, mikrokristallinem Wachs, synthetischen Fasern, natürlichen Fasern und Mischungen hiervon.

10. Verfahren zur Herstellung der Gebiss-Haftzusammensetzung gemäß mindestens einem der Ansprüche 1 bis 9, umfassend die Schritte:
a) teilweises Neutralisieren eines C₁-C₄-Alkylvinylether-Maleinsäure-Copolymeren mit einem oder mehreren Metallkationen, gewählt aus zweiwertigen Metallkationen, einwertigen Metallkationen und Mischungen hiervon;
b) Zugeben von Eisen(III)-Kationen zu dem teilweise neutralisierten Copolymer zur Bildung einer Partialcopolymersalzzusammensetzung; und
c) Trocknen der Zusammensetzung zur Bildung eines Pulvers.

11. Verfahren nach Anspruch 10, umfassend weiterhin die Schritte:
a) Mischen des Pulvers mit Wasser und/oder einem oder mehreren Lösungsmittelen zur Bildung einer Mischung; und
b) Aufbringen der Mischung auf eine nichtadhäsive, selbsttragende Schicht.

## Revendications

1. Composition adhésive pour prothèse dentaire, comprenant un sel partiel d'un copolymère d'alkyl(en C₁-C₄)vinyléther/acide maléique, dans laquelle le sel partiel comprend une fonction sel cationique constituée essentiellement de:
a) 0,01% à 10% de cations de fer ferrique; et
b) 0,1% à 75% de cations de métaux divalents et/ou monovalents choisis parmi le zinc, le calcium, le magnésium, le potassium, le sodium, l'ammonium et leurs mélanges,
dans laquelle les cations de strontium ne sont pas utilisés en combinaison avec les cations de zinc.

2. Composition adhésive pour prothèse dentaire selon la revendication 1, dans laquelle les cations de métaux divalents et/ou monovalents sont choisis parmi le strontium, le calcium, le magnésium, le potassium, le sodium, l'ammonium et leurs mélanges.

3. Composition adhésive pour prothèse dentaire selon la revendication 1 ou la revendication 2, dans laquelle les cations de métaux sont des cations de métaux divalents chosis parmi le zinc, le calcium, le magnésium et leurs mélanges.

4. Composition adhésive pour prothèse dentaire selon l'une quelconque des revendications 1 à 3, comprenant 0,5% à 5% de cations de fer ferrique.

5. Composition adhésive pour prothèse dentaire selon l'une quelconque des revendications 1 à 4, comprenant en outre un composant adhésif supplémentaire choisi parmi les gommes naturelles, les polymères synthétiques, les polymères mucoadhésifs, les polymères hydrophiles, les polymères naturels, les dérivés de saccharide, les dérivés de cellulose, et leurs mélanges.

6. Composition adhésive pour prothèse dentaire selon l'une quelconque des revendications 1 à 5, comprenant un dérivé de cellulose adhésif choisi parmi l'hydroxyéthylcellulose, l'hydroxypropylcellulose, l'hydroxypropylméthylcellulose, la carboxyméthylcellulose sodique, et leurs mélanges.

7. Composition adhésive pour prothèse dentaire selon l'une quelconque des revendications 1 à 6, comprenant en outre un arôme choisi parmi le menthol, le lactate de menthyle, l'essence de menthe poivrée, l'essence de menthe verte, l'alcool de feuille, les p-menthane-carboxamides, et leurs mélanges.

8. Composition adhésive pour prothèse dentaire selon l'une quelconque des revendications 1 à 7, comprenant 10% à 50% de carboxyméthylcellulose, en poids de la composition, dans laquelle la fonction sel cationique est essentiellement constituée de:
a) 0,1% à 3% de cations de fer ferrique;
b) 10% à 65% de zinc, et
c) 10% à 75% de calcium,
des groupes carboxyle initiaux ayant réagi.

9. Composition adhésive pour prothèse dentaire selon l'une quelconque des revendications 1 à 8, comprenant en outre au moins une couche non adhésive autoporteuse choisie parmi le polyester, le polypropylène, le nylon, la rayonne, le poly(oxyde d'éthylène), l'acétate de cellulose, les dérivés de cellulose, un tissu, un molleton fibreux, du papier, du plastique, du cuir, de la cire microcristalline, des fibres synthétiques, des fibres naturelles, et leurs mélanges.

10. Procédé pour la préparation de la composition adhésive pour prothèse dentaire selon l'une quelconque des revendications 1 à 9, comprenant les étapes consistant à:
a) neutraliser partiellement un compolymère d'alkyl(en C₁-C₄)-vinyléther/acide maléique avec un ou plusieurs cations de métaux choisis parmi les cations de métaux divalents, les cations de métaux monovalents, et leurs mélanges;
b) ajouter des cations fer ferrique au copolymère partiellement neutralisé pour former une composition de sel partiel de copolymère; et
c) sécher la composition pour former une poudre.

11. Procédé selon la revendication 10, comprenant en outre les étapes consistant à
a) mélanger la poudre avec de l'eau et/ou un ou plusieurs solvants pour créer un mélange; et
b) appliquer le mélange sur une couche autoporteuse non adhésive.
